# EUROPÄISCHE PATENTANMELDUNG

(11) **EP 1 024 133 A2**
(43) Veröffentlichungstag der Anmeldung: **02.08.2000**
(21) Anmeldenummer: 00101758.1
(22) Anmeldetag: 28.01.2000
(51) Int. Cl.: C07C 209/62, C07C 227/20, C07K 1/06, C07K 1/12, C07D 263/44, C08G 69/00

(54) **Verfahren zur Decarbamoylierung von N-cabamoylgeschützten Verbindungen, neue Carbonsäureanhydride und deren Verwendung**

(30) Priorität: 28.01.1999 DE 19903268
(71) Anmelder: Degussa-Hüls Aktiengesellschaft, 60287 Frankfurt am Main (DE)
(72) Erfinder: Collet, Hélène, 9 rue de Nazareth, 34000 Montpellier (FR); Mion, Louis, 34080 Montpellier (FR); Taillades, Jacques, 34170 Castelmau Le Lez (FR); Commeyras, Auguste, 34830 Clapiers (FR); Barenschee, Ernst Robert, Dr., 07076 Scotch Plains, NJ (US); Knaup, Günther, Dr., 63486 Bruchköbel (DE)

(57) **Zusammenfassung**

Verfahren zur Decarbamoylierung von N-carbamoylgeschützten Verbindungen, neue Carbonsäureanhydride und deren Verwendung.

Die Erfindung richtet sich auf ein Verfahren zur Decarbamoylierung von N-carbamoylgeschützten Verbindungen der allgemeinen Formel (I) 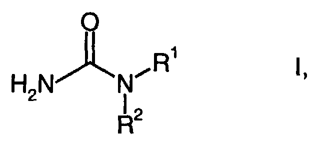 mittels Stickoxiden der allgemeinen Formel (II)

NₓO_{y} II.

Durch diese Reaktion können neue vormals nicht herstellbare Carbonsäureanhydride der allgemeinen Formel (III) 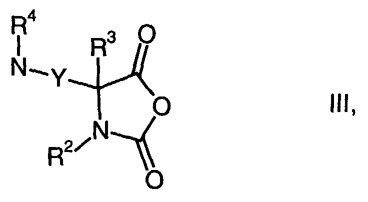 generiert werden.

Verwendung der erfindungsgemäßen Anhydride in der Synthese bioaktiver Wirkstoffe und zur Herstellung von Polyaminosäuren.

## Beschreibung

Die vorliegende Erfindung betrifft ein Verfahren zur Decarbamoylierung von N-carbamoylgeschützten Verbindungen der allgemeinen Formel (I) 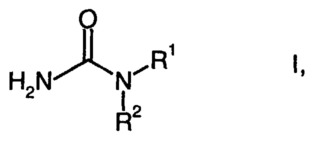 mit Stickoxiden der allgemeinen Formel (II)

NₓO_{y} II.

Weiterhin richtet sich die Erfindung auf neue Produkte der allgemeinen Formel (III) 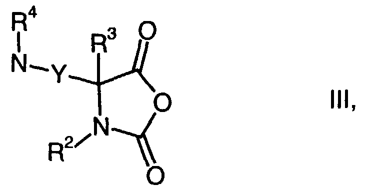 sowie deren Säureadditionssalze und deren Verwendung.

Amine und insbesondere Aminosäuren und Peptide sind wichtige Substrate zur Herstellung von bioaktiven Wirkstoffen. Zudem handelt es sich bei diesen Verbindungen oft um chirale Moleküle, so dass es im überwiegenden Maße gewünscht ist, möglichst enantiomerenreine Verbindungen zu handhaben. Methoden, die es gestatten, Amingruppen enthaltende Verbindungen wie z. B. Aminosäuren bzw. Peptide höchst stereokonservativ herzustellen bzw. umzusetzen, sind deshalb stets willkommen. Dazu gehören unter anderen Methoden zum Einführen und Abspalten von Schutzgruppen.

Enantiomerenangereicherte N-Carbamoylaminosäuren lassen sich darüber hinaus sehr einfach durch enzymatische Spaltung von racemischen Hydantoinen (DE 195 29 211.1) herstellen. Darüber hinaus ist die Carbamoylgruppe eine N-Schutzgruppe, die relativ leicht z. B. durch Reaktion von primären oder sekundären Aminen mit Cyanaten eingeführt werden kann. Obwohl diese Verfahren im Vergleich zu anderen Schutzgruppen wie z. B. Benzyloxycarbonyl (Z) oder tert.-Butyloxycarbonyl (Boc) verhältnismäßig kostengünstig und einfach sind, wurde diese Gruppe bisher nur selten eingesetzt. Der Grund dafür ist, dass zur Abspaltung entweder eine Nitrosierung mit einem NO⁺-Donor, z. B. mit Natriumnitrit und Salzsäure, oder eine Carbamoylase eingesetzt werden mußte (PCT/EP96/02782).

Bei der ersten Variante ist, insbesondere bei komplexeren Molekülen, mit einer Reihe von Nebenreaktionen sowie mit einem großen Salzanfall zu rechnen. Bei der enzymatischen Abspaltung ist die Struktur der Substrate sehr begrenzt und die entsprechenden Enzyme sind z. T. nur schwer zugänglich.

Aus der US 5,777,076 ist bekannt, daß man N-Carbamoylaminosäuren mit gasförmigem NO und Sauerstoff in Lösung zu N-Carbonsäureanhydriden umsetzten kann. Als Nebenprodukte dieser Reaktion entstehen lediglich Stickstoff und Wasser. Arbeitet man in wäßriger Lösung, so entsteht in untergeordnetem Maße etwas freie Aminosäure.

Aufgabe der Erfindung war es daher, ein Verfahren zu entwickeln, das eine einfache, breit anwendbare und kostengünstige Abspaltung der Carbamoylschutzgruppe ermöglicht.

Diese Aufgabe wird durch ein Verfahren mit den kennzeichnenden Merkmalen des Anspruchs 1 erreicht. Besondere Ausgestaltungen des Verfahrens sind in den Ansprüchen 2 bis 10 unter Schutz gestellt.

Die Ansprüche 11 - 13 stellen erstmals durch dieses Verfahren herstellbare erfindungsgemäße Verbindungen unter Schutz, Anspruch 14 und 15 dagegen bevorzugte Anwendungen.

Dadurch, daß zur Decarbamoylierung von N-carbamoylgeschützten Verbindungen der allgemeinen Formel (I) 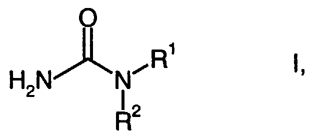 worin
R¹ (C₁-C₈)-Alkyl, (C₂-C₈)-Alkenyl, (C₂-C₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-c₁₉)-Heteroalkyl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl, (C₃-C₈)-Cycloalkenyl, (C₁-C₈)-Alkyl-(C₃-C₈)-Cycloalkenyl, (C₃-C₈)-Cycloalkenyl-(C₁-C₈)-Alkyl, (C₆-C₈)-Cycloalkinyl, (C₁-C₈)-Alkyl-(C₆-C₈)-Cycloalkinyl, (C₆-C₈)-Cycloalkinyl-(C₁-C₈)-Alkyl bedeuten kann, wobei die oben genannten Reste einfach oder mehrfach mit Heteroatomen wie Hal, N, P, O, S substituiert sein können, oder wobei eine oder mehrere CH₂-Gruppen durch Heteroatome wie N, P, O, S ausgetauscht sein können, oder
R¹ bildet mit dem anhängenden Stickstoffatom eine Aminosäure, oder
R¹ bildet mit dem anhängenden Stickstoffatom den N-Terminus eines Peptids,
R² Wasserstoff, R¹ bedeuten kann,
   Stickoxiden der allgemeinen Formel (II)

   NₓO_{y} II,

   worin x die Zahl 1 und y die Werte 1 oder 2 oder x die Zahl 2 und y die Werte 1, 2 oder 3 besitzen kann, eingesetzt werden, und man die Umsetzung als Gas-Feststoffreaktion, vorzugsweise im Wirbelbett, durchführt, gelangt man völlig überraschend und damit in nicht weniger vorteilhafter Weise zu den entschützten Derivaten der allgemeinen Formel (I). Durch Eintragen der geschützten Derivate (I) in einen Gasstrom, welcher die oben angegebenen nitrosen Gase ggf. in Gegenwart weiterer Inertgase (N₂, He, Ar, etc.) enthält, wird ohne Umweg über eine aufwendige Kristallisation und Trocknung, wie sie bei einer Decarbamoylierung in Lösung notwendig ist, gleich das fertige Produkt erhalten. Das Verfahren ist deshalb im technischen Maßstab sehr einfach umzusetzen, liefert sehr hohe Ausbeuten und trägt somit zur Senkung der Produktionskosten bei. Die aus der Decarbamoylierung der Verbindungen der allgemeinen Formel I resultierenden Amine werden in Form ihrer Säureadditionssalze erhalten. Im Falle, daß keine weiteren Säuren zugesetzt werden, sind dies überwiegend die Salze der Salpetersäure, d. h. die Nitrate.

Im Prinzip können als Gase zur Entcarbamoylierung alle nitrosen Gase verwendet werden, welche befähigt sind, die Stickstoffatome der Carbamoylverbindung zu nitrosieren. Es kommen somit Stickoxide wie NO als auch NO₂ bzw. deren Dimere oder Mischungen derselben in Frage. Bevorzugt setzt man für diesen Zweck Verbindungen der allgemeinen Formel (II) ein, in denen x = 1 und y = 2 ist. Gleichsam sind auch Gase aktiv, bei denen Verbindungen der allgemeinen Formel (II) mit x = 1 und y = 1 oder x = 2 und y = 1 eingesetzt werden, wobei die Reaktionen dann in Gegenwart von Sauerstoff ablaufen.

Für die Reaktion kommen alle diejenigen Verbindungen in Frage, welche über eine sekundäre oder primäre N-carbamoylgeschützte Aminogruppe verfügen. Besonders bevorzugt sind geschützte sekundäre und primäre Amine, Peptide sowie Aminosäuren. Hinsichtlich der Reaktion lassen sich auch N,N'-dicarbamoylgeschützte Diaminosäuren einsetzen. Im Falle von N,N'-Dicarbamoyl-L-lysin beispielsweise erhält man reines L-Lysin-N-Carbonsäureanhydrid, welches auf anderem Wege bisher nicht herstellbar war.

Um die Decarbamoylierung zu beschleunigen, können prinzipiell alle dem Fachmann geläufigen Additive zur Reaktion beigemengt werden. Bevorzugt fügt man dem zu entschützenden Feststoff bei der Gas-Feststoffreaktion soviel Wasser hinzu, daß keine flüssige Phase entsteht. Bevorzugt setzt man 0,5 - 50 Gew.-%, äußerst bevorzugt 5 - 20 Gew.-%, Wasser, bezogen auf den eingesetzten Feststoff, zu.

Zur weiteren Beschleunigung der Reaktion kann darüber hinaus auch 0,01 - 20 Gew.-%, vorzugsweise 0,1 - 10 Gew.-%, bezogen auf den eingesetzten Feststoff, einer Säure, vorzugsweise eine organische Carbonsäure, wie z. B. Essigsäure, Propionsäure, Buttersäure, Halogencarbonsäuren wie z. B. Mono-, Di- oder Trichloressigsäure, Mono-, Di- oder Tribromessigsäure, Mono-, Di- oder Trifluoressigsäure, eine organische Sulfonsäure wie z. B. Methansulfonsäure, Toluolsulfonsäure, oder eine anorganische Säure wie z. B. Salzsäure, Bromwasserstoffsäure, Schwefelsäure oder Phosphorsäure der Reaktion zugesetzt werden. Die Säuren können entweder direkt als Flüssigkeit (z. B. Essigsäure, Trifluoressigsäure) oder in Form einer wäßrigen Lösung zugesetzt werden. Besonders bevorzugt ist die Zugabe sowohl von Wasser als auch von Säure.

Die Wahl der Temperatur während der Decarbamoylierung ist einerseits abhängig von der Stabilität der Substrate, andererseits von dem Temperaturpunkt, ab dem eine annehmbar schnelle Reaktion beobachtet werden kann. Weiterhin sollte die Temperatur über den Siedepunkten der eingesetzten Stickoxide der allgemeinen Formel (II) liegen. Im allgemeinen liegen die Temperaturen bei der Reaktion zwischen -20 - 150 °C, vorzugsweise 10 - 50 °C.

Ein weiterer Aspekt der vorliegenden Erfindung betrifft neue Produkte der allgemeinen Formel (III) 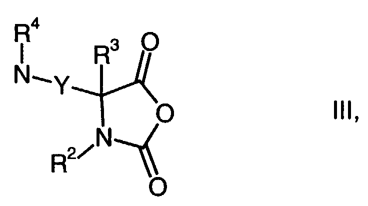 sowie deren Säureadditionssalze,
worin
R² die oben angegebene Bedeutung annehmen kann und
R³ und R⁴ unabhängig voneinander H, (C₁-C₈)-Alkyl, (C₂-C₈)-Alkenyl, (C₂-C₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroalkyl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl, (C₃-C₈)-Cycloalkenyl, (C₁-C₈)-Alkyl-(C₃-C₈)-Cycloalkenyl, (C₃-C₈)-Cycloalkenyl-(C₁-C₈)-Alkyl, (C₆-C₈)-Cycloalkinyl, (C₁-C₈)-Alkyl-(C₆-C₈)-Cycloalkinyl, (C₆-C₈)-Cycloalkinyl-(C₁-C₈)-Alkyl bedeuten können, wobei die oben genannten Reste einfach oder mehrfach mit Heteroatomen wie Hal, N, P, O, S substituiert sein können, oder wobei eine oder mehrere CH₂-Gruppen durch Heteroatome wie N, P, O, S ausgetauscht sein können, und
Y für einen linearen oder verzweigten (C₁-C₈)-Alkylenrest oder (C₇-C₁₉)-Arylalkylenrest, welche gegebenenfalls mehrfach mit (C₁-C₈)-Alkyl substituiert sein können, steht, wobei die oben genannten Reste einfach oder mehrfach mit Heteroatomen wie Hal, N, P, O, S substituiert sein können, oder wobei eine oder mehrere CH₂-Gruppen durch Heteroatome wie N, P, O, S ausgetauscht sein können.

Besonders bevorzugt sind Verbindungen der allgemeinen Formel (III), bei denen R², R³ und R⁴ für H stehen und Y einen linearen oder verzweigten (C₁-C₈)-Alkylenrest darstellt. Ganz besonders bevorzugt sind Verbindungen der allgemeinen Formel (III), die ausgehend von Lysin erhalten werden.

Die Verbindungen der allgemeinen Formel (III) und deren Säureadditionssalze sind nach Verfahren des Standes der Technik (H. R. Kriecheldorf, "α-Amino acid-N-Carboxy-Anhydrides and Related Heterocycles", Springer Verlag Berlin, 1987, S. 1 - 51) nicht herstellbar, da sie sofort inter- oder intramolekular weiterreagieren. Durch Anwendung der äußerst milden Decarbamoylierungsbedingungen bei diesem Gas/Feststoff-Verfahren gelangt man hingegen völlig überraschend zu den gewünschten Derivaten.

Die Verbindungen der allgemeinen Formel (III) werden nach dem erfindungsgemäßen Verfahren direkt in Form ihrer Säureadditionssalze erhalten, die keine inter- oder intramolekularen Weiterreaktionen eingehen. Im Falle, daß keine weiteren Säuren zugesetzt werden, sind dies überwiegend die Salze der Salpetersäure, d. h. die Nitrate.

Bevorzugt werden die mit diesem Verfahren darstellbaren Verbindungen der allgemeinen Formel (III) als Intermediate in der organischen Synthese, bevorzugt zur Synthese bioaktiver Wirkstoffe, z. B. Aminosäureamiden und Dipeptiden, eingesetzt. Weiterhin sind diese N-Carbonsäureanhydride geeignete Ausgangsprodukte zur Herstellung von Polyaminosäuren. Die Polykondensation kann dabei nach bekannten Methoden (H. R. Kriecheldorf, "α-Amino acids-N-Carboxy-Anhydrides and Related Heterocycles", Springer Verlag Berlin, 1987, S. 59 - 132) durchgeführt werden. Die Verbindungen der allgemeinen Formel (III) können dabei entweder selbst, gegebenenfalls mit anderen Zusätzen, polymerisiert werden, oder mit NCA's anderer Aminosäuren zu Copolymerisaten umgesetzt werden.

Ein Gebiet der organischen Synthese, bei denen das erfindungsgemäße Verfahren äußerst vorteilhaft anwendbar ist, ist die Abspaltung der Carbamoylgruppe aus N-Carbamoylpeptiden. Diese können z. B. gemäß der PCT/EP96/02782 einfach durch enzymatische Verknüpfung hergestellt werden. Bei diesen Verbindungen verläuft die erfindungsgemäße Schutzgruppenabspaltung quantitativ und ohne daß die peptidische Amidbindung angegriffen wird.

Besonders vorteilhaft ist, dass die Entschützungsprozedur so mild verläuft, dass enantiomerenangereicherte Substrate ohne Racemisierung abreagieren.

Unter dem Begriff Aminosäuren versteht man im Rahmen der Erfindung organische Verbindungen, welche sowohl mindestens eine Carbonsäuregruppe als auch mindestens eine Aminogruppe im Molekül besitzen. Vorzugsweise sind dies die gesamte Gruppe der proteinogenen Aminosäuren. Es werden jedoch auch nicht proteinogene Aminosäuren wie z. B. α-Amino-adipinsäure, α-Amino-buttersäure, γ-Amino-buttersäure, Anthranilsäure, ε-Amino-capronsäure, 1-Amino-cyclohexancarbonsäure, 1-Amino-cyclopentancarbonsäure, α-Amino-isobuttersäure, β-Amino-isobuttersäure, β-Alanin, Amino-malonsäure, 1-Amino-cyclopropancarbonsäure, Azetidincarbonsäure, Aziridincarbonsäure, 4-Chlorphenylalanin, α,γ-Diamino-buttersäure, α,β-Diaminopropionsäure, 3,4-Dihydroxy-phenylalanin, 2,2-Dimethyl-1,3-thiazolidin-4-carbonsäure, 4-Fluor-phenylalanin, Homocystein, Homoserin, δ-Hydroxylysin, 4-Hydroxy-phenylglycin, 4-Hydroxy-prolin, 3-Hydroxy-prolin, Isoserin, Isovalin, Lanthionin, 2-Naphthylalanin, Neopentylglycin, Norleucin, Norvalin, Oxylysin, Ornithin, Pipecolinsäure, Penicillamin, Phenylglycin, Picolinsäure, β-Phenylserin, Pyridylalanin, Chinoxalincarbonsäure, Sarkosin, Thiazolidin-4-carbonsäure, tert.-Leucin dazugezählt.

Unter einem (C₁-C₁₈)-Alkylrest wird im Rahmen der Erfindung ein Rest mit 1 bis 18 gesättigten C-Atomen verstanden, der beliebige Verzweigungen aufweisen kann. Insbesondere sind unter diese Gruppe die Reste Methyl, Ethyl, n-Propyl, Isopropyl, n-Butyl, Isobutyl, sec-Butyl, tert-Butyl, Pentyl, Hexyl, etc. subsumierbar. Ein (C₁-C₈)-Alkylrest beschreibt den eben definierten Rest im Umfang von 1 bis 8 C-Atomen.

Ein (C₂-C₁₈)-Alkenylrest weist die für den (C₁-C₁₈)-Alkylrest genannten Merkmale auf, wobei innerhalb des Restes mindestens eine Doppelbindung vorhanden sein muß.

Ein (C₂-C₁₈)-Alkinylrest weist die für den (C₁-C₁₈)-Alkylrest genannten Merkmale auf, wobei innerhalb des Restes mindestens eine Dreifachbindung vorhanden sein muß.

Unter einem (C₆-C₁₈)-Arylrest wird ein aromatischer Rest mit 6 bis 18 C-Atomen verstanden. Insbesondere zählen hierzu Reste wie Phenyl-, Naphthyl-, Anthryl-, Phenanthryl-, Biphenylreste.

Ein (C₇-C₁₉)-Aralkylrest ist ein über einen (C₁-C₈)-Alkylrest an das Molekül gebundener (C₆-C₁₈)-Arylrest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Reste angesehen, wie 1-, 2-, 3-Furyl, wie 1-, 2-, 3-Pyrrolyl, 1-,2-, 3-Thienyl, 2-, 3-, 4-Pyridyl, 2-, 3-, 4-, 5-, 6-, 7-Indolyl, 3-, 4-, 5-Pyrazolyl, 2-,4-, 5-Imidazolyl, Acridinyl, Chinolinyl, Phenanthridinyl, 2-, 4-, 5-, 6-Pyrimidinyl.

Unter einem (C₄-C₁₉)-Heteroaralkyl wird ein dem (C₇-C₁₉)-Aralkylrest entsprechendes heteroaromatisches System verstanden.

Mithin bezeichnet im Rahmen der Erfindung ein (C₃-C₈)-Cycloalkylrest einen Rest der Gruppe der cyclischen Alkylreste mit 3 bis 8 C-Atomen und ggf. beliebiger Verzweigung. Insbesondere sind unter diese Gruppe die Reste Cyclopropyl, Cyclobutyl, Cyclopentyl, Cyclohexyl, Cycloheptyl, Cyclooctyl zu subsumieren.

Ein (C₃-C₈)-Cycloalkenylrest ist ein (C₃-C₈)-Cycloalkylrest mit einer oder mehreren Doppelbindungen im Ringsystem. Ein (C₆-C₈)-Cycloalkinyl ist dementsprechend ein (C₆-C₈)-Cycloalkylrest mit einer Dreifachbindung im Ringsystem.

Unter Hai versteht man Fluor, Chlor, Brom, Iod.

Unter dem Begriff "mit Heteroatomen wie N, O, P, S substituiert" ist zu verstehen, daß ggf. einer oder mehrere Reste organischen Ursprungs wie z. B. R¹ oder Wasserstoff über ein wie eben genanntes Heteroatom an den entsprechenden Substituenten gebunden sein kann.

Die dargestellten Strukturen und Formeln beziehen sich bei Vorliegen eines oder mehrerer stereogener Zentren auf die einzelnen Enantiomere und/oder Diastereomere sowie auf die racemischen Derivate.

Unter dem Begriff enantiomerenangereichert wird im Rahmen der Erfindung der Anteil eines Enantiomers im Gemisch mit seiner optischen Antipode in einem Bereich von >50 % und <100 % verstanden.

Die folgenden Beispiele sollen die Erfindung erläutern.

### 1. Decarbamoylierung von N-Carbamoyl-L-leucyl-glycin

Zu festem N-Carbamoyl-L-leucyl-glycin, das ggf. mit Wasser oder Trifluoressigsäure vermengt wurde, werden in einem verschlossenen 50 ml Kolben unter Inertgasatmosphäre mittels einer Spritze die gasförmigen Reaktanden (NO, NO₂ und Sauerstoff) gegeben. Nach 30 min wird Stickstoff durchgeleitet und der erhaltene Feststoff mittels ¹H-NMR Spektren in DMSO untersucht, wobei die charakteristischen Signale des Produktes bei 8.15 ppm (NH₂₋Gruppe) und 8.88 ppm (NH-Gruppe) sowie die des Eduktes bei 5.55 ppm (NH₂-Gruppe), 6.12 und 8.25 (2 NH-Gruppen) ausgewertet werden.

Die Ergebnisse der Versuche sind in der folgenden Tabelle zusammengefaßt:

| **Nr.** | **Temp. [°C]** | **Edukt [mg]** | **NO [ml]** | **O**_{**2**} **[ml]** | **NO**_{**2**} **[ml]** | **H**_{**2**}**O [**µ**l]** | **TFA-OH[** µ**l]** | **Ausb. [%]** |
|---|---|---|---|---|---|---|---|---|
| **1** | 18 | 25 | 6 | 2.4 | - | - | | 24 |
| **2** | 18 | 25 | 6 | 2.4 | - | 2 | - | 97 |
| **3** | 18 | 25 | 6 | 2.4 | - | - | 1 | 100 |
| **4** | 18 | 25 | | | 3 | | - | 14 |
| **5** | 18 | 25 | | | 3 | 2 | - | 25 |
| **6** | 28 | 25 | | | 3 | - | - | 14 |
| **7** | 28 | 25 | | | 3 | 4 | - | 64 |
| **8** | 18 | 25 | - | - | 4 | 2 | - | 35 |
| **9** | 28 | 25 | - | - | 4 | - | - | 21 |
| **10** | 28 | 25 | - | - | 4 | 4 | - | 100 |
| **11** | 18 | 25 | - | - | 5 | 0 | - | 34 |
| **12** | 18 | 25 | - | - | 5 | 2 | - | 70 |
| **13** | 28 | 25 | - | - | 5 | | - | 48 |
| **14** | 28 | 25 | - | - | 5 | 4 | - | 100 |
| **16** | 28 | 25 | - | - | 5 | 6 | - | 100 |

### 2. Decarbamoylierung von N-Carbamoyl-L-alanyl-L-phenylalanin

20 mg N-Carbamoyl-L-alanyl-L-phenylalanin werden analog dem Beispiel 1 mit 12 ml NO und 4,8 ml Sauerstoff umgesetzt. Nach dem ¹H-NMR-Spektrum besteht der erhaltene Feststoff zu 40 % aus L-Alanyl-L-phenylalaninhydronitrat.

### 3. Decarbamoylierung von N-Carbamoyl-benzylamin

29 mg N-Carbamoyl-benzylamin werden analog dem Beispiel 1 mit 12 ml NO und 4,8 ml Sauerstoff bei 28°C umgesetzt. Nach dem ¹H-NMR-Spektrum erhält man einen quantitativen Umsatz.

### 4. Decarbamoylierung von N,N'-Dicarbamoyl-L-lysin

21.3 mg N,N'-Dicarbamoyl-L-lysin werden analog Beispiel 1 mit 7.5 ml NO₂ und 6 ml Wasser bei 28°C umgesetzt. Nach dem ¹H-NMR-Spektrum besteht der erhaltene Feststoff aus reinem L-Lysin-N-carbonsäureanhydridhydronitrat.
¹H-NMR (DMSO) : 1.0 - 1. 9 (m, 6H, (CH₂)₃), 2,8 (m, 2H, CH₂-N), 4,45 (m, lH, CH), 7,0 - 8,0 (s, 3H, NH₃), 9,20 (s, 1H, NH).

## Patentansprüche

1. Verfahren zur Decarbamoylierung von N-carbamoylgeschützten Verbindungen der allgemeinen Formel (I) 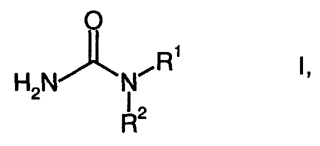 worin
R¹ bedeutet (C₁-C₈)-Alkyl, (C₂-C₈)-Alkenyl, (C₂-C₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroalkyl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl, (C₃-C₈)-Cycloalkenyl, (C₁-C₈)-Alkyl-(C₃-C₈)-Cycloalkenyl, (C₃-C₈)-Cycloalkenyl-(C₁-C₈)-Alkyl, (C₆-C₈)-Cycloalkinyl, (C₁-C₈)-Alkyl-(C₆-C₈)-Cycloalkinyl, (C₆-C₈)-Cycloalkinyl-(C₁-C₈)-Alkyl, wobei die oben genannten Reste einfach oder mehrfach mit Heteroatomen wie Hal, N, P, O, S substituiert sein können, oder wobei eine oder mehrere CH₂-Gruppen durch Heteroatome wie N, P, O, S ausgetauscht sein können, oder
R¹ bildet mit dem anhängenden Stickstoffatom eine Aminosäure oder
R¹ bildet mit dem anhängenden Stickstoffatom den N-Terminus eines Peptids,
R² Wasserstoff, R¹ bedeuten kann, mit Stickoxiden der allgemeinen Formel (II)
NₓO_{y} II,
worin x die Zahl 1 und y die Werte 1 oder 2 oder x die Zahl 2 und y die Werte 1, 2 oder 3 besitzen kann,
**dadurch gekennzeichnet,**
dass die Umsetzung als Gas-Feststoffreaktion, vorzugsweise im Wirbelbett, stattfindet.

2. Verfahren nach Anspruch 1,
**dadurch gekennzeichnet,**
dass in der Verbindung der allgemeinen Formel (II) x = 1 und y = 2 ist.

3. Verfahren nach Anspruch 1,
**dadurch gekennzeichnet,**
dass in der Verbindung der allgemeinen Formel (II) x = 1 und y = 1 ist und zusätzlich Sauerstoff eingesetzt wird.

4. Verfahren nach Anspruch 1,
**dadurch gekennzeichnet,**
dass in der Verbindung der allgemeinen Formel (II) x = 2 und y = 1 ist und zusätzlich Sauerstoff eingesetzt wird.

5. Verfahren nach einem oder mehreren der Ansprüche 1 bis 4,
**dadurch gekennzeichnet,**
dass man ein N-carbamoylgeschütztes Peptid oder eine N-carbamoylgeschützte Aminosäure als Verbindung der allgemeinen Formel (I) einsetzt.

6. Verfahren nach einem oder mehreren der Ansprüche 1 bis 5,
**dadurch gekennzeichnet,**
dass man eine N,N'-dicarbamoylgeschützte Diaminocarbonsäure als Verbindung der allgemeinen Formel (I) einsetzt.

7. Verfahren nach Anspruch 6,
**dadurch gekennzeichnet,**
dass man eine N,N'-Dicarbamoyl-lysin als Verbindung der allgemeinen Formel (I) einsetzt.

8. Verfahren nach einem oder mehreren der Ansprüche 1 bis 7,
**dadurch gekennzeichnet,**
dass der Umsetzung 0,5 - 50 Gew.-%, vorzugsweise 5 - 20 Gew.-%, Wasser, bezogen auf den eingesetzten Feststoff, zugesetzt werden.

9. Verfahren nach einem oder mehreren der Ansprüche 1 bis 8,
**dadurch gekennzeichnet,**
dass der Umsetzung 0,01 - 20 Gew.-%, vorzugsweise 0,1 - 10 Gew.-%, einer Säure, vorzugsweise Trifluoressigsäure, bezogen auf den eingesetzten Feststoff, zugesetzt werden.

10. Verfahren nach einem oder mehreren der Ansprüche 1 bis 9,
**dadurch gekennzeichnet,**
dass die Umsetzung bei einer Temperatur von -20 - 150 °c, vorzugsweise 10 - 50 °C, stattfindet.

11. Verbindungen der allgemeinen Formel (III) 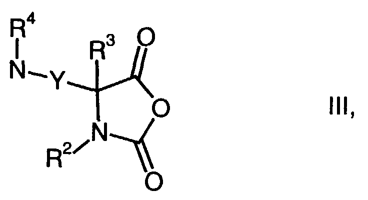 sowie deren Säureadditionssalze,
worin
R² die oben angegebene Bedeutung annehmen kann und
R³ und R⁴ unabhängig voneinander H, (C₁-C₈)-Alkyl, (C₂-C₈)-Alkenyl, (C₂-C₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroalkyl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl, (C₃-C₈)-Cycloalkenyl, (C₁-C₈)-Alkyl-(C₃-C₈)-Cycloalkenyl, (C₃-C₈)-Cycloalkenyl-(C₁-C₈)-Alkyl, (C₆-C₈)-Cycloalkinyl, (C₁-C₈)-Alkyl-(C₆-C₈)-Cycloalkinyl, (C₆-C₈)-Cycloalkinyl-(C₁-C₈)-Alkyl bedeuten kann, wobei die oben genannten Reste einfach oder mehrfach mit Heteroatomen wie Hai, N, P, O, S substituiert sein können, oder wobei eine oder mehrere CH₂-Gruppen durch Heteroatome wie N, P, O, S ausgetauscht sein können, und
Y für einen linearen oder verzweigten (C₁-C₈)-Alkylenrest oder (C₇-C₁₉)-Arylalkylenrest, welche gegebenenfalls mehrfach mit (C₁-C₈)-Alkyl substituiert sein können, steht, wobei die oben genannten Reste einfach oder mehrfach mit Heteroatomen wie Hal, N, P, O, S substituiert sein können, oder wobei eine oder mehrere CH₂-Gruppen durch Heteroatome wie N, P, O, S ausgetauscht sein können.

12. Verbindungen nach Anspruch 11 bei denen R², R³ und R⁴ für H stehen und Y einen linearen oder verzweigten (C₁-C₈)-Alkylenrest darstellt.

13. Verbindungen nach Anspruch 12, die ausgehend von Lysin erhalten werden.

14. Verwendung der Verbindungen nach Anspruch 11 in der Synthese von bioaktiven Wirkstoffen.

15. Verwendung der Verbindungen nach Anspruch 11 in einem Verfahren zur Herstellung von Polyaminosäuren.
